# EUROPÄISCHE PATENTANMELDUNG

(11) **EP 2 455 414 A1**
(43) Veröffentlichungstag der Anmeldung: **23.05.2012**
(21) Anmeldenummer: 10191481.0
(22) Anmeldetag: 17.11.2010
(51) Int. Cl.: C08G 63/08, C08G 63/80, C08G 63/90

(54) **Verfahren zur Herstellung von hochmolekularen, resorbierbaren Polyestern, die mit dem Verfahren herstellbaren hochmolekularen resorbierbaren Polyester und deren Verwendung**

(71) Anmelder: Evonik Röhm GmbH, 64293 Darmstadt (DE)
(72) Erfinder: Bocionek, Dirk, 55218 Ingelheim am Rhein (DE); Enderla, Anja, 55437 Ockenheim (DE)

(57) **Zusammenfassung**

Die Erfindung betrifft ein Verfahren zur Herstellung von hochmolekularen, resorbierbaren Polyestern durch Massepolymerisation und anschließende Reinigung des erhaltenen Rohpolymers sowie die mit dem Verfahren herstellbaren hochmolekularen, resorbierbaren Polyester, wobei durch gezielte Auswahl des Kettenlängenmoderators die Eigenschaften der Polyester eingestellt werden können. Die hochmolekularen, resorbierbaren Polyester finden insbesondere im medizinischen oder pharmazeutischen Bereich Verwendung. **Figur 1****:** Veranschaulichung eines Ablaufschemas für die Durchführung der Polymerisation gemäß dem erfindungsgemäßen Verfahren (Verfahrensschritte (a) bis (f)) anhand einer beispielhaften Ausführungsform

## Beschreibung

Die Erfindung betrifft ein Verfahren zur Herstellung von hochmolekularen, resorbierbaren Polyestern durch Massepolymerisation und anschließende Reinigung des erhaltenen Rohpolymers sowie die mit dem Verfahren herstellbaren hochmolekularen, resorbierbaren Polyester und deren Verwendung.

### HINTERGRUND DER ERFINDUNG

### TECHNISCHES GEBIET

Aus resorbierbaren Polymeren hergestellte medizinische Produkte stellen aufgrund ihrer vielfältigen Anwendungsmöglichkeiten in der modernen Medizin und in Zusammenhang mit den Fortschritten in der technischen Entwicklung eine wichtige Alternative zu konventionellen medizinischen Anwendungen dar. Es handelt sich um hochtechnologische Produkte, die als Resultat jahrelanger Forschung und Entwicklung erhalten werden.

Resorbierbare Polymere gemäß der vorliegenden Erfindung sind aliphatische Polyester in Form von Homopolymeren, insbesondere auf der Basis von Lactiden (L-Lactid, D-Lactid, D,L-Lactid, meso-Lactid) oder Glycoliden, oder in Form von Copolymeren mit zwei oder mehr unterschiedlichen Comonomereinheiten, insbesondere auf Basis der genannten Monomeren.

Resorbierbare Polyester werden ohne weiteres im Körper zu den monomeren Verbindungen abgebaut, die dann nach Metabolisierung oder in unveränderter Form ausgeschieden werden. Das Abbauprodukt von Polylactid ist beispielsweise Milchsäure, die ein Zwischenprodukt im Kohlenhydratabbau darstellt und in allen Geweben enthalten ist. Die L-Form der Milchsäure wird im Citrat-Zyklus zu Wasser und Kohlendioxid abgebaut, die D-Form wird in nicht-metabolisierter Form ausgeschieden. Polyglycolid wird über Glycolsäure und weiter durch Glycolat-Oxidase und die Transaminase zu Glycin metabolisiert.

Die resorbierbaren Polyester sind auch mit anderen abbaubaren Monomeren kombinierbar oder können durch Zusatz von Hilfsstoffen modifiziert werden.

Die Herstellung der Polyester kann über eine Ringöffnungs-Polymerisation erfolgen, die eine Gleichgewichtsreaktion darstellt. Diese wird anhand der nachfolgenden Reaktionsgleichungen veranschaulicht:
(a) ringöffnende Polymerisation eines Lactids (L-Lactid, D-Lactid, D,L-Lactid, meso-Lactid) zum Homopolymer: 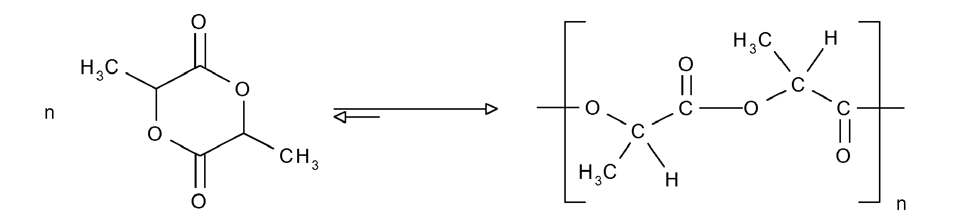
(b) ringöffnende Polymerisation eines Lactids und eines Glycolids zum Copolymer: 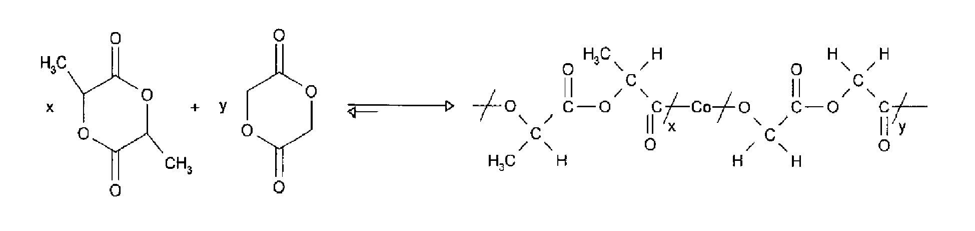 wobei n jeweils die Gesamtzahl der wiederkehrenden Einheiten darstellt und x und y die jeweilige Anzahl der Bausteine angibt.

Die cyclischen Anhydride werden als Ausgangsmaterialien beispielsweise aus den entsprechenden Hydroxycarbonsäuren unter entwässernder Kondensation und Bildung eines cyclischen Dimers nach Ringschluss erhalten. Ein Lactid ist ein cyclisches Dimer aus Milchsäure bzw. 2-Hydroxypropansäure, ein Glycolid ist ein cyclisches Dimer aus Glycolsäure bzw. Hydroxyessigsäure. Das Lactid kann in D-, L-, D,L- oder meso-Form vorliegen. Das D-Lactid resultiert aus der D-Milchsäure, das L-Lactid wird aus der L-Milchsäure erhalten, das meso-Lactid ergibt sich als cyclisches Dimer aus D,L-Milchsäure und das D,L-Lactid stellt eine racemische Mischung aus D-Lactid und L-Lactid dar.

Bei der Ringöffnungs-Polymerisation lassen sich zwei Verfahrensvarianten unterscheiden: die gerührte Schmelzepolymerisation und die nicht-gerührte Massepolymerisation. Letztere zeichnet sich durch mildere Reaktionsbedingungen und längere Reaktionszeiten aus. Ab einer bestimmten Kettenlänge lassen sich die zu bildenden Polymere bei den Umsetzungstemperaturen nicht mehr rühren, so dass diese Polymere praktisch nicht mehr im gerührten Verfahren hergestellt werden können. Daher wird erfindungsgemäß die Massepolymerisation eingesetzt. Durch die milderen Reaktionsbedingungen bei der Massepolymerisation kommt es typischerweise zu einer gegenüber der Schmelzepolymerisation deutlich geringeren Verfärbung der Rohpolymere, vor allem im Fall von glycolidhaltigen Polymeren.

Polyester mit hohem Molekulargewicht sind insbesondere dann von Interesse, wenn längere Abbauzeiten des Polymers erwünscht sind.

Durch Auswahl der Monomereinheiten der Polyester können die Eigenschaften gezielt eingestellt und insbesondere auf die verschiedenen Anwendungsbereiche maßgeschneidert werden. Reines Poly(L-lactid) ist z.B. teilkristallin; Poly(D,L-lactid) und dessen Copolymere liegen in amorpher Form vor. Derartige amorphe Polymere weisen den Vorteil einer guten Löslichkeit in organischen Lösungsmitteln, beispielsweise in Aceton, Chloroform oder Dichlormethan, auf. Reines Poly(L-lactid) ist beispielsweise nicht in Aceton, sondern nur in halogenierten Lösungsmitteln löslich. Die amorphen Polymere sind daher von Vorteil, da auf halogenierte Lösungsmittel völlig verzichtet werden kann. Die gute Löslichkeit ist außerdem vorteilhaft für die Herstellung oder auch Verarbeitung dieser Polymere, beispielsweise wenn hieraus Produkte hergestellt werden sollen, wobei die Verarbeitung der Polymere über einen Lösungsschritt erfolgen muss. Dies ist beispielsweise bei der Herstellung von Mikrokapseln der Fall. Diese Polymere werden daher insbesondere für die Herstellung von pharmazeutischen Formulierungen verwendet. Der Einsatzbereich von Copolymeren auf Basis von Milch- und Glycolsäure ist demnach vorteilhafterweise die Herstellung von Wirkstoffkapseln zur kontrollierten Freisetzung pharmazeutischer Wirksubstanzen.

Homopolymere aus Milchsäure (Poly(L-lactide)) kommen hauptsächlich für die Produktion resorbierbarer medizinischer Implantate im menschlichen oder tierischen Körper zum Einsatz, wie beispielsweise als Material für Fixationselemente, Folien, Membranen oder auch pharmazeutische Freisetzungssysteme.

Aus dem Stand der Technik sind resorbierbare Polyester bereits bekannt geworden:
So beschreiben J. Dahlmann und G. Rafler in der Veröffentlichung "Biodegradable polymers, 7th comm., On the mechanism of ring-opening polymerization of cyclic esters of aliphatic hydroxycarboxylic acids by means of different tin compounds" aus Acta Polymer, 44, Seiten 103-107, 1993, den Einfluss von Zinnverbindungen als Katalysatoren auf die Ringöffnungspolymerisation von Lactiden und Lactonen, insbesondere am Beispiel von D,L-Lactid. Die untersuchten Zinnverbindungen waren z.B. Zinncarboxylate, wie Zinn(II)diacetat und Zinn(II)dioctanoat und Tetraorganostannane, wie Zinn(II)tetraphenyl. Die Polymerisation wurde unter Stickstoff in einem Dreihalskolben durchgeführt, wobei zunächst die Monomeren geschmolzen, anschließend die Zinnverbindung und, wenn notwendig, weitere Verbindungen, zugegeben wurden. Zur Entfernung von Restmonomeren wurde das Produkt in Chloroform gelöst und bei Raumtemperatur mit Methanol gefällt. Eine Erhöhung der Katalysatormenge führte zu einer Erhöhung der Polymerisationsgeschwindigkeit und Zunahme des Polymerisationsgrades. Auch die Zugabe einer Verbindung mit einer alkoholischen OH-Gruppe, wie Milchsäureethylester, wurde untersucht und es wurde gefunden, dass diese Verbindungen bei der Polymerisation des D,L-Lactids zu einer Abnahme des Polymerisationsgrads, abhängig von der eingesetzten Menge, führen.

Die EP 0 624 613 A2 offenbart ein Verfahren zur Herstellung von Polyestern durch eine Ringöffnungs-Polymerisation einer cyclischen Esterverbindung in Gegenwart einer Hydroxylverbindung als Molekulargewichtsregulator, wobei das Molekulargewicht des Polyesters genau in dem gewünschten Bereich eingestellt werden kann, indem die Menge an freier Carbonsäure in der cyclischen Esterverbindung möglichst genau abgeschätzt und hieraus die Menge an einzusetzender Hydroxylverbindung abgeleitet wird. Als Hydroxylverbindung werden Alkohole, Hydroxycarbonsäuren und Saccharide genannt. Die Abschätzung der Menge an freier Carbonsäure erfolgt über die Messung der elektrischen Leitfähigkeit der cyclischen Esterverbindung in Lösung. Die Polymerisation, z.B. zu Polyglycolsäure, wird bei sehr hohen Temperaturen von etwa 235°C durchgeführt.

Das US-Patent 5,502,158 bezieht sich auf abbaubare Materialien, die nicht-toxische hydrolytisch abbaubare Polymere und nicht-toxische Modifizierungsmittel enthalten. Das Polymer ist typischerweise Polymilchsäure. Als Modifizierungsmittel finden Verwendung: Dicarbonsäuren und deren Derivate, Polyester von Dicarbonsäuren, Tricarbonsäuren und deren Derivate, Polyester von Tricarbonsäuren, cyclische Diester von alpha-Hydroxycarbonsäuren und deren Derivate und Oligomere, beta-, delta, gamma- und epsilon-Lactone, Oligomere von alpha-Hydroxycarbonsäuren, Ester von Oligomeren von alpha-Hydroxycarbonsäuren, Benzoesäurederivate, Epoxyderivate, Glykolderivate, Phthalsäurederivate, Phosphorsäurederivate, Ketone, Amide, Nitrile und deren Kombinationen. Es wird beschrieben, dass die Modifizierungsmittel bekanntermaßen dazu dienen, im Polymer den Glasumwandlungspunkt (oder die Glasumwandlungstemperatur) abzusenken. Es werden insbesondere Filme, Formprodukte, Laminate, Schäume, Pulver, Non-wovens, Kleber und Beschichtungen bereitgestellt, die nach Gebrauch recycelt werden können.

Schließlich beschreibt das US-Patent 6,362,308 B1 Copolymere aus Lactid und Glycolid mit hohem Glycolidanteil sowie ein Verfahren zur Herstellung des Copolymers, wonach eine Mischung, umfassend D,L-Lactid, Glycolid, eine Hydroxycarbonsäure und Zinnoctoat auf eine Temperatur zwischen etwa 175 und 200°C erhitzt wird, um das Copolymer herzustellen.

Die Praxis hat gezeigt, dass die bekannten resorbierbaren Polyester in Einzelfällen nicht in hinreichendem Maße über das gewünschte Eigenschaftsprofil verfügen und Nachteile aufweisen. So ist bekannt, dass die thermischen Eigenschaften dieser Polymere, wie die Glasumwandlungstemperatur, neben ihrer amorphen Struktur maßgeblich von der Kettenlänge abhängen. Je kürzer die Kettenlänge und damit je niedermolekularer das Polymer desto geringer ist die Glasumwandlungstemperatur. Auch bei hochmolekularen Polymeren hat der eingesetzte Kettenlängenregler durch die Endgruppen Einfluss auf die chemischen und thermischen Eigenschaften des Polymers. Ein Kettenregler (auch bezeichnet als Co-Katalysator) dient zur Einstellung der gewünschten Kettenlänge der zu polymerisierenden Masse, bindet kovalent an das Kettenende und beeinflusst hierdurch die Eigenschaften des hergestellten Polyesters, und zwar um so mehr je kürzer die Polymerkette ist. Beispielweise zeigen Polymere mit gleichen Polymerketten aber unterschiedlichen Kettenenden einen unterschiedlichen Glasumwandlungspunkt. Die Höhe des Glasumwandlungspunkts spielt eine Rolle für die Formstabilität bei Lagerung und damit die Lagerstabilität der Polyester. Bei amorphen Copolymeren aus D,L-Lactid und Glycolid liegt der Glasumwandlungspunkt, abhängig vom Molekulargewicht, in einem Temperaturbereich zwischen ca. 35 und 50°C. Bei Polyestern aus nicht glycolidhaltigen Polymeren liegt der Glasumwandlungspunkt etwas höher. Bei einer Temperatur oberhalb des Glasübergangs erweichen diese Materialien und sind nicht mehr formstabil. Der wesentliche Nachteil der bekannten resorbierbaren Polyester ist somit, dass sie bei der Anwendungstemperatur im Körper von 37°C, und teilweise schon bei üblichen Lager-und/oder Transporttemperaturen keine Formstabilität aufweisen. Daraus ergibt sich ein erhöhter Aufwand bei Lagerung und Transport der bekannten resorbierbaren Polyester, d.h. temperaturkontrollierte Lagerung und Transport sind zwingend erforderlich, und es resultieren deutlich eingeschränkte Verwendungsmöglichkeiten.

Auch andere Eigenschaften der Polyester, wie beispielsweise Verkapselungs- und Freisetzungseigenschaften, werden durch die Kettenenden beeinflusst.

Weitere Nachteile bislang bekannter Polyester ergeben sich aus den bekannten Herstellungsverfahren. Die ringöffnende Polymerisation, zum Beispiel von D,L-Lactid und/oder Glycolid, stellt eine Gleichgewichtsreaktion dar, so dass in den Rohpolymerisaten stets ein Restgehalt an nicht umgesetzten Monomeren vorhanden ist. Der Einsatz im pharmazeutischen und medizinischen Bereich erfordert jedoch die Einhaltung eines extrem hohen Qualitätsniveaus, wobei derartige Restgehalte in keinem Fall toleriert werden können. Im Hinblick auf den vorgesehenen Verwendungszweck im menschlichen oder tierischen Körper und auch unter dem Aspekt, Produkte mit reproduzierbaren Eigenschaften hieraus herzustellen, müssen die Restmonomere daher auf ein Mindestmaß herabgesenkt bzw. nahezu vollständig entfernt werden. Hierzu sind in der Regel aufwändige Aufarbeitungen erforderlich, um die hohe Qualität sicherzustellen. Beispielsweise müssen zusätzliche separate Reinigungs- und Aufarbeitungsverfahren durchgeführt werden.

Ein weiterer Nachteil der im Polymer enthaltenen Restmonomeren ist, dass diese als Weichmacher fungieren und somit den Glasumwandlungspunkt in unerwünschtem Maße weiter absenken. Nachteilig ist weiterhin, dass Restmonomere generell den Abbau katalysieren und damit beschleunigen.

Aufgrund der oben geschilderten Nachteile und bekannten wertvollen Eigenschaften von hochmolekularen, resorbierbaren Polyestern besteht demnach ein Bedarf, weitere hochmolekulare, resorbierbare Polyester bereitzustellen, welche die Nachteile aus dem Stand der Technik nicht aufweisen und deren Eigenschaften gezielt an die gewünschten Anwendungen angepasst werden können. Weiterhin soll auch ein Herstellungsverfahren für die hochmolekularen, resorbierbaren Polyester zur Verfügung gestellt werden, das als industriell anwendbares Verfahren zur Herstellung von hochmolekularen, resorbierbaren Polyestern geeignet ist, bevorzugt bei gemäßigten Temperaturen arbeitet und welches es gestattet hochmolekulare, resorbierbare Polyester in hoher Qualität und in großem Maßstab herzustellen.

### KURZE ZUSAMMENFASSUNG DER ERFINDUNG

Die Erfindung betrifft ein Verfahren zur Herstellung von hochmolekularen, resorbierbaren Polyestern durch Massepolymerisation und anschließende Reinigung des erhaltenen Rohpolymers sowie die mit dem Verfahren herstellbaren hochmolekularen, resorbierbaren Polyester, wobei durch gezielte Auswahl des Kettenlängenmoderators die Eigenschaften der Polyester eingestellt werden können. Gegenstand der Erfindung ist auch eine Vorrichtung zur Durchführung des Verfahrens sowie die Verwendung der erfindungsgemäß hergestellten hochmolekularen, resorbierbaren Polyester im medizinischen und pharmazeutischen Bereich.

### DETAILLIERTE BESCHREIBUNG DER ERFINDUNG

Ein erster Aspekt der vorliegenden Erfindung betrifft ein Verfahren zur Herstellung von hochmolekularen, resorbierbaren Polyestern, umfassend die Schritte
(a) Aufschmelzen von Monomeren in einem Aufschmelzgefäß;
(b) Zugabe eines Katalysators;
(c) Zugabe eines Kettenlängenmoderators, der ausgewählt ist aus der Gruppe bestehend aus Hydroxydicarbonsäuren und, Hydroxytricarbonsäuren;
(d) Homogenisieren der Reaktionsmischung;
(e) Überführen der Reaktionsmischung in ein oder mehrere Reaktionsbehälter;
(f) Durchführen der Polymerisationsreaktion in dem einen oder mehreren Reaktionsbehälter(n) bis zum erwünschten Umsetzungsgrad der Polymerisation;
(g) Lösen des erhaltenen Rohpolymers in Aceton;
(h) Ausfällen des Polymers durch Zugeben von Wasser;
(i) Abtrennen des erhaltenen Polymers;
(j) gegebenenfalls Zerkleinern des erhaltenen Polymers und
(k) Trocknen des Polymers.

Nach dem erfindungsgemäßen Verfahren werden zunächst in Schritt (a) die Monomeren geschmolzen. Vorzugsweise wird unter inerter Atmosphäre gearbeitet, insbesondere bei besonders hohen Molekulargewichten ist eine inerte Atmosphäre zweckmäßig. Die Temperatur und Dauer hierfür hängen von den ausgewählten Monomeren ab. Die Monomeren können ausgewählt werden aus jeder Art von Monomeren, welche nach Polymerisation zu einem Polyester führen. Bevorzugt sind solche Monomere, welche durch ringöffnende Polymerisation der entsprechenden cyclischen Monomeren polymerisiert werden können. Bevorzugt sind daher cyclische Esterverbindungen. Exemplarische cyclische Esterverbindungen, die erfindungsgemäß als Monomere zum Einsatz kommen können sind: Glycolid, Lactid, wie beispielsweise L-Lactid, D-Lactid, D,L-Lactid, meso-Lactid, beta-Propiolacton, gamma-Butyrolacton, delta-Valerolacton, epsilon-Caprolacton, 3-Methyl-1,4-dioxa-2,5-dion, p-Dioxanon, Morpholin-2,5-dion, Morpholin-2-on und Mischungen dieser voranstehend genannten Monomere.

Ein weiterer Gegenstand der vorliegenden Erfindung betrifft das voranstehend genannte Verfahren zur Herstellung von resorbierbaren Polyestern, dadurch gekennzeichnet, dass die verwendeten Monomere ausgewählt sind aus der Gruppe bestehend aus Glycolid, L-Lactid, D-Lactid, D,L-Lactid, meso-Lactid, beta-Propiolacton, gamma-Butyrolacton, delta-Valerolacton, epsilon-Caprolacton, 3-Methyl-1,4-dioxa-2,5-dion, p-Dioxanon, Morpholin-2,5-dion, Morpholin-2-on und Mischungen dieser voranstehend genannten Monomere.

Ein weiterer Gegenstand der vorliegenden Erfindung betrifft das voranstehend genannte Verfahren zur Herstellung von resorbierbaren Polyestern, dadurch gekennzeichnet, dass die verwendeten Monomere ausgewählt sind aus der Gruppe bestehend aus Glycolid, L-Lactid, D-Lactid, D,L-Lactid, meso-Lactid und Mischungen dieser Monomere.

Während des gesamten Verfahrens wird vorzugsweise der über der Reaktionsmasse befindliche Raum mittels eines inerten und wasserfreien Gases inertisiert. Bevorzugt werden hierfür Argon, Helium oder Stickstoff verwendet, besonders bevorzugt ist Stickstoff.

Das Aufschmelzen der Monomeren (eine Monomerverbindung) oder Monomerenmischung (mehrere verschiedene Monomerverbindungen) findet in einem Aufschmelzgefäß vorzugsweise unter Rühren statt. Je nach Maßstab kann in einem kleinen Gefäß, zum Beispiel im Labor, oder auch in großindustriellem Maßstab gearbeitet werden. Vorzugsweise wird in einem Rührwerksreaktor gearbeitet, dessen Größe je nach dem gewünschten Ansatz ausgewählt wird.

Als Rührwerksreaktor zum Aufschmelzen und Homogenisieren der Reaktionsmischung kann beispielsweise ein konventioneller Reaktor eingesetzt werden, dessen Innenwandung mit einem gegenüber der Reaktionsmischung chemisch inerten Werkstoff, beispielsweise Edelstahl, Glas, Emaille oder Hastelloy oder einem ähnlichen Material ausgekleidet ist. Die bevorzugte Größe des Reaktors richtet sich nach der gewünschten Chargengröße und kann zweckmäßigerweise in einem Bereich von etwa 2 bis 100 Litern liegen.

Für die Polymerisation wird ein Katalysator eingesetzt. Als Katalysatoren können beispielsweise Verbinudngen verwendet werden, die ausgewählt sind aus der Gruppe bestehend aus Zinnverbindungen, wie beispielsweise Zinnchlorid, Zinnoxid, Zinnfluorid, Tetraphenylzinn, Zinnoctanoat, Zinnacetat, Zinnstearat, Zink oder Zinkverbindungen, wie beispielsweise metallisches Zink, Zinkoxid, Zink-L-lactat, Zinkstearat, ZnCl₂, ZnBr₂ oder Znl₂, Antimonverbindungen, wie beispielsweise Antimontrioxid, Antimontrifluorid, herkömmliche Wismuth-, Blei-, Arsen-, Bor- oder Phosphorverbindungen, wie beispielsweise Wismutnitrat, Bleioxid, Bleistearat, Bortrifluorid, Tetraethylammoniumborid, Tributylarsin, Tributylphosphin, Triethylamin, Tributylamin oder andere katalytisch wirksame Verbindungen. Besonders bevorzugt sind Zinnverbindungen, ganz besonders bevorzugt ist Zinn(II)ethylhexanoat.
Es können auch Mischungen von Katalysatoren eingesetzt werden.

Die Menge an Katalysator hängt von den Monomeren, der Art des ausgewählten Katalysators, den gewählten Verfahrensbedingungen, wie Temperatur, Reaktionsdauer, Polymerisationsgrad und dergleichen ab. Beim erfindungsgemäßen Verfahren werden die Katalysatoren bevorzugt in geringen Konzentrationen eingesetzt, um durch eine geringe Reaktionsgeschwindigkeit einerseits die Wärmeentwicklung während der Polymerisation zu minimieren und andererseits zu verhindern, dass die Reaktionsmasse bereits im Aufschmelzgefäß nennenswert polymerisiert, wodurch aufgrund des Viskositätsanstiegs das Überführen in die Reaktionsbehälter erschwert werden würde. Weiterhin ist der Einsatz nur geringer Katalysatormengen im Hinblick auf den Einsatz der Polyester im menschlichen Körper vorteilhaft. Im Falle von Zinnverbindungen liegen die bevorzugten Konzentrationen bei etwa 200 ppm oder weniger, bevorzugt bei etwa 100 ppm oder weniger, bevorzugter bei etwa 75 ppm oder weniger, ganz besonders bevorzugt bei etwa 60 ppm oder weniger, insbesondere bei etwa 50 ppm oder weniger (jeweils berechnet als Zinn, bezogen auf die gesamte Reaktionsmasse).

Die Zugabe des Katalysators gemäß Verfahrensschritt (b) kann als reine Substanz oder vorzugsweise als Lösung in einem inerten und physiologisch unbedenklichen Verdünnungsmittel erfolgen. Bevorzugte Verdünnungsmittel sind aliphatische oder aromatische Kohlenwasserstoffe, insbesondere Toluol oder Xylol.

Gemäß Verfahrensschritt (c) wird ein Kettenlängenmoderator zugegeben. Kettenlängenmoderatoren, die zur Regulierung der Kettenlänge eingesetzt werden, können prinzipiell alle Arten von Alkoholen darstellen. Daneben können auch Verbindungen verwendet werden, die neben der alkoholischen Hydroxylgruppe eine oder mehrere weitere funktionelle Gruppe tragen. Somit können die chemischen Eigenschaften des Kettenendes gezielt verändert werden.

Ein weiterer Gegenstand der vorliegenden Erfindung betrifft das voranstehend genannte Verfahren zur Herstellung von resorbierbaren Polyestern, dadurch gekennzeichnet, dass als Kettenlängenmoderator eine Verbindung verwendet wird, die ausgewählt ist aus der Gruppe bestehend aus Hydroxycarbonsäuren, Hydroxydicarbonsäuren, Hydroxytricarbonsäuren oder Hydroxycarbonsäureestern.

Ein weiterer Gegenstand der vorliegenden Erfindung betrifft das voranstehend genannte Verfahren zur Herstellung von resorbierbaren Polyestern, dadurch gekennzeichnet, dass als Kettenlängenmoderator eine Verbindung verwendet wird, die ausgewählt ist aus der Gruppe bestehend aus Hydroxydicarbonsäuren und Hydroxytricarbonsäuren.

Die verwendeten Hydroxycarbonsäuren können ausgewählt sein aus der Gruppe bestehend aus Milchsäure, Glycolsäure oder Hydroxycapronsäure.
Die verwendeten Hydroxydicarbonsäuren können ausgewählt sein aus der Gruppe bestehend aus Äpfelsäure (2-Hydroxybernsteinsäure) oder Weinsäure (2,3-Dihydroxybernsteinsäure).
Die verwendete Hydroxytricarbonsäure kann ausgewählt sein aus der Gruppe bestehend aus Zitronensäure.
Die verwendeten Hydroxycarbonsäureester können ausgewählt sein aus der Gruppe bestehend aus Milchsäureester, Glycolsäureester oder Hydroxycapronsäureester, wobei die entsprechenden Ethylester dieser Verbindungen, wie beispielsweise Milchsäureethylester, Glycolsäureethylester und Hydroxycapronsäureethylester, bevorzugt verwendet werden.

Ein weiterer Gegenstand der vorliegenden Erfindung betrifft das voranstehend genannte Verfahren zur Herstellung von resorbierbaren Polyestern, dadurch gekennzeichnet, dass als Kettenlängenmoderator Äpfelsäure oder Zitronensäure verwendet wird.

Es können auch Mischungen der voranstehend genannten Kettenlängenmoderatoren zum Einsatz kommen.

Äpfelsäure und Zitronensäure sind erfindungsgemäß besonders bevorzugt, weil hierdurch besonders bevorzugte Polyester erhalten werden können. In überraschender Weise kann der Einsatz von diesen speziellen Kettenlängenmoderatoren zu einer Erhöhung des Glasumwandlungspunkts bzw. der Glasumwandlungstemperatur herangezogen werden. Wie bereits ausgeführt hat die Erhöhung des Glasumwandlungspunkts bei den erfindungsgemäßen Polyestern besonders große Vorteile für den geplanten Einsatz im menschlichen oder tierischen Körper, beispielsweise in Form von Implantaten oder Fixationsmaterialien oder pharmazeutischen Formulierungen.

Die erfindungsgemäßen Polyester müssen eine ausreichende mechanische Festigkeit bzw. bei den pharmazeutischen Formulierungen eine ausreichende Formstabilität mit einer gewünschten Hydrolysierbarkeit bzw. Abbaubarkeit für den gewünschten Verwendungszweck vereinen, bei pharmazeutischen Formulierungen die gewünschte Verkapselungs- bzw. Freisetzungsrate erreichen und ihre Form und Festigkeit für eine vordefinierte Zeitdauer behalten, um erst anschließend, je nach Anforderung, relativ schnell oder relativ langsam abgebaut zu werden. Aufgrund des erhöhten Glasumwandlungspunkts durch entsprechende Auswahl des Kettenlängenmoderators, kann demnach besser gewährleistet werden, dass das Polyestermaterial auch im menschlichen oder tierischen Körper seine Form und Festigkeit für die gewünschte Zeitdauer behält.

Weiterhin sind die erfindungsgemäßen Polyester auch während der Verarbeitung und als zu verarbeitende Formulierungen, zum Beispiel in Form von Mikropartikeln, ausreichend formstabil.

Ein weiterer Vorteil der erfindungsgemäßen Polyester ist, dass durch Vorliegen einer oder mehrerer freier Carboxylgruppen im Polymer die Hydrophilie des Polymers erhöht werden kann, was Auswirkungen auf den Abbau und die Freisetzung von Wirkstoffen hat. Besonders vorteilhaft sind als Kettenlängenmoderatoren Hydroxydicarbonsäuren oder Hydroxytricarbonsäuren, welche gegenüber einfachen Carbonsäuren eine höhere Säurezahl aufweisen. Hierdurch können besonders nützliche Eigenschaften in den hergestellten Polyestern erzeugt werden, wie beispielsweise eine gesteigerte Wasseraufnahme der Polyester.

Die Auswahl der jeweiligen Endgruppen und somit eine Erhöhung der Bindungsstellen kann zur Modifizierung der Eigenschaften der erfindungsgemäßen Polyester herangezogen werden, da es hierdurch z.B. gelingt insbesondere bei Verwendung in Form von pharmazeutischen Formulierungen die Menge an enthaltenem Wirkstoff ("Verkapselungsrate") zu modifizieren, d.h. die Menge an enthaltenem Wirkstoff in gewünschter Weise zu erhöhen oder abzusenken. Weiterhin kann durch die ausgewählten Endgruppen auf die Art der Freisetzung, insbesondere das Freisetzungsprofil, des Wirkstoffs gezielt Einfluss genommen werden.

Die geschilderten vorteilhaften Eigenschaften der Polyester können erreicht werden, ohne dass auf andere Polymerbestandteile zurückgegriffen werden müsste, wodurch die Abbaubarkeit wieder beeinträchtigt werden könnte.

Durch den Einsatz verschiedener Kettenlängenmoderatoren können Polyester mit unterschiedlichen Endgruppen an einem Kettenende erhalten werden. Diese unterschiedlichen Kettenenden sind erfindungsgemäß insbesondere bevorzugt eine oder mehrere freie Carboxylgruppen (Milchsäure, Glycolsäure, Äpfelsäure oder Zitronensäure als Kettenlängenmoderator) oder Alkylester-Endgruppen (Milchsäureethylester als Kettenlängenmoderator). Selbstverständlich können auch andere Kettenlängenmoderatoren zum Einsatz kommen. Zur Bestimmung der jeweiligen Endgruppe kann beispielsweise der Säurewert anhand einer Titration bestimmt werden und es können gegebenenfalls zusätzlich ¹H-NMR-Spektren aufgenommen werden.

Die bevorzugte Konzentration des Kettenlängenmoderators hängt von der Struktur des Moderators und vom gewünschten Molekulargewicht des Polymeren ab und liegt vorzugsweise im Bereich von 0 bis etwa 20.000 ppm, bevorzugter im Bereich von 0 bis etwa 15.000 ppm, besonders bevorzugt im Bereich von 0 bis etwa 10.000 ppm, insbesondere im Bereich von etwa 50 bis etwa 8.500 ppm, jeweils bezogen auf die gesamte Reaktionsmasse.

Der hier verwendete Begriff "etwa" bedeutet innerhalb ±20%, bevorzugt innerhalb ±10% und noch bevorzugter innerhalb ±5% eines vorgegebenen Werts oder Bereichs.

Die Zugabe von Katalysator erfolgt stets nach dem Schmelzen der Monomere. Der Kettenlängenmoderator kann vor, während oder nach dem Aufschmelzen der Monomere zugegeben werden. Bevorzugt werden nach dem Aufschmelzen der Monomeren erst der Katalysator und dann der Kettenlängenmoderator zugegeben. Die Reihenfolge der Verfahrensschritte (b) und (c) kann auch vertauscht werden; die Zugabe des Kettenlängenmoderators erfolgt dann vor der Zugabe des Katalysators. Der Kettenlängenmoderator kann auch bereits zu den Monomeren in das Reaktionsgefäß gegeben und zusammen mit den Monomeren geschmolzen und anschließend erst der Katalysator zugegeben werden. Katalysator und Kettenlängenmoderator können auch gleichzeitig zugegeben werden, dies erfolgt dann nach dem Aufschmelzen der Monomeren. Nach einer weiteren bevorzugten Ausführungsform der Erfindung kann der Katalysator auch im Kettenlängenmoderator gelöst werden und beide können zusammen zur Reaktionsmischung nach dem Aufschmelzen der Monomeren zugegeben werden; in diesem Fall wird als Kettenlängenmoderator bevorzugt Dodecanol verwendet.

Gemäß Verfahrensschritt (d) wird die Reaktionsmischung homogenisiert. Dies kann beispielsweise unter Durchmischen, vorzugsweise durch Rühren oder Schütteln, erreicht werden.

Im Anschluss daran wird die Reaktionsmasse vom Aufschmelzgefäß in ein oder mehrere Reaktionsbehälter überführt (Verfahrensschritt (e)). Dies kann beispielsweise mittels konventioneller Pumpen, durch Schwerkraft oder auch durch Druckaufgabe mittels Inertgas, insbesondere Stickstoff, auf das Aufschmelzgefäß, z.B. einen Aufschmelzreaktor, erfolgen. Zur Entfernung potentiell vorhandener partikulärer Verunreinigungen aus der Schmelze kann optional ein Schmelzefilter, vorzugsweise ausgewählt aus Kunststoff oder Edelstahl, zwischengeschaltet werden. Dies ist nicht in jedem Fall erforderlich.

Erfindungsgemäß bevorzugt werden die Reaktionsbehälter hinsichtlich ihres Volumens kleiner ausgewählt als das Aufschmelzgefäß. Das Füllvolumen des Reaktionsbehälters wird vorzugsweise derart gewählt, dass es in einem Bereich von etwa 0.5 bis etwa 5 Litern liegt, insbesondere etwa 1 Liter beträgt. In der Regel liegt das Verhältnis des Innenvolumens des Aufschmelzgefäßes, wie einem Rührwerksreaktor, zum Innenvolumen eines der Reaktionsbehälter im Bereich von etwa 200 : 1 bis etwa 2 : 1, vorzugsweise im Bereich von etwa 100 : 1 bis etwa 2 : 1.

Für das Verfahren lassen sich Reaktionsbehälter aus beliebigem Material einsetzen, welche unter den Reaktionsbedingungen, insbesondere bei den gewählten Reaktionstemperaturen, chemisch und thermisch stabil sind. Bevorzugt werden Behälter aus ein oder mehreren Kunststoffen eingesetzt, besonders bevorzugt Kunststoffe der Gruppe der Polyolefine, Polycarbonate, fluorierte und teilfluorierte Kunststoffe. Besonders bevorzugt werden Polypropylen, Polytetrafluorethan (Teflon^{®}) und Polymethylpenten (PMP) verwendet.

Die Polymerisationsreaktion wird in der Regel bei einer Temperatur im Bereich von etwa 100°C bis etwa 160°C durchgeführt, in Einzelfällen kann dieses Temperaturintervall aber auch verlassen werden. Die Reaktion wird bevorzugt isotherm durchgeführt.

Die erforderlichen Reaktionszeiten hängen von der Reaktivität des oder der Monomeren, der gewählten Temperatur und Katalysatorkonzentration und vom erforderlichen Umsetzungsgrad ab. Bevorzugt werden Reaktionszeiten im Bereich von etwa 0.5 bis etwa 10 Tagen, besonders bevorzugt im Bereich von etwa 2 bis etwa 8 Tagen, insbesondere etwa 3 bis etwa 7 Tagen eingehalten. Diese Reaktionszeiten sind jedoch nur beispielhaft und können auch über- oder unterschritten werden.

Die Polymerisation (Verfahrensschritt (f)) wird in dem oder den Reaktionsbehältern bis zum gewünschten Umsetzungsgrad, d.h. Polymerisationsgrad, durchgeführt. In der Regel ist der gewünschte Polymerisationsgrad erreicht, wenn von den eingesetzten Monomeren weniger als 10 Gew.-%, vorzugsweise im Bereich von 0.5 Gew.-% bis 5 Gew.-%, im erfindungsgemäß hergestellten Polymer enthalten sind.

Nach der Polymerisation kann das Polymer aus dem Reaktionsbehälter entnommen werden und für jeden einzelnen Reaktionsbehälter kann getrennt die Aufarbeitung bzw. Reinigung erfolgen oder es können auch die Produkte aus mehreren Reaktionsbehältern zusammengeführt und dann gereinigt werden. Das aus der Polymerisation erhaltene Rohpolymer wird dann zur Reinigung in Aceton gelöst (Verfahrensschritt (g)). Gegebenenfalls kann die erhaltene Lösung zur Entfernung potentiell vorhandener partikulärer Verunreinigungen zusätzlich filtriert werden. Der Filter ist für diesen Zweck vorzugsweise aus Kunststoff oder Edelstahl ausgewählt. Es können aber auch andere Filter Verwendung finden. Das Filtrieren wird bevorzugt durchgeführt, ist aber nicht in jedem Fall erforderlich.

Durch Zugabe eines Überschusses an Wasser wird das gelöste Polymer ausgefällt, während die Monomeren in Lösung bleiben und somit aus dem Polymer entfernt werden können (Verfahrensschritte (h) und (i)). Die Menge an Aceton und zuzusetzendem Wasser ergibt sich aus der gewählten Ansatzmenge und kann vom Fachmann anhand des Stands der Technik ohne weiteres durch einige wenige, orientierende Versuch bestimmt werden.

Die Reinigung des erhaltenen Rohpolymers durch Lösen in Aceton, gegebenenfalls Filtrieren der Lösung und dann Ausfällen des Polymers aus der Lösung mit Wasser kann auch mehrfach wiederholt werden, bis der gewünschte Reinigungsgrad erreicht wird.

Zusätzlich kann nach dem Abtrennen des ausgefällten Polymers ein oder mehrmals mit Wasser gewaschen werden.

Bevorzugt wird das ausgefällte Polymer kontinuierlich aus der Lösung entfernt. Dies kann beispielsweise mittels eines rotierenden Siebs oder auch in anderer Weise erfolgen.

Das Trocknen des gereinigten Polymers (Verfahrensschritt (k)) kann in üblicher Weise, beispielsweise in einem Wirbelschichttrockner oder einer anderen Trocknungsvorrichtung, gegebenenfalls nach einem zusätzlichen Zerkleinern des Polymers, durchgeführt werden.

Das erhaltene gereinigte Polymer weist einen deutlich verringerten Restmonomergehalt auf, der auch unterhalb der Nachweisgrenze liegen kann. Ein Restgehalt von weniger als etwa 0.5 Gew.-%, vorzugsweise 0.2 bis 0.4 Gew.%, ist jedoch in der Regel bereits ausreichend, um die gewünschten Qualitätsanforderungen für den pharmazeutischen oder medizinischen Bereich bereitzustellen.

Gegenstand der Erfindung ist auch eine Vorrichtung zur Durchführung des erfindungsgemäßen Verfahrens, wobei die obigen Ausführungen zum Verfahren gleichermaßen für die erfindungsgemäße Vorrichtung gelten.

Die vorliegende Erfindung bezieht sich ebenfalls auf die hochmolekularen, resorbierbaren Polyester, die gemäß dem erfindungsgemäßen Verfahren herstellbar sind, wobei der Kettenlängenmoderator ausgewählt ist aus einer Hydroxydicarbonsäure, bevorzugt Äpfelsäure (2-Hydroxybernsteinsäure), oder einer Hydroxytricarbonsäure, bevorzugt Zitronensäure.

Mit dem erfindungsgemäßen Verfahren herstellbare Polyester sind entweder Homopolyester oder Copolyester, je nach den ausgewählten Monomeren. Bevorzugte erfindungsgemäße Homopolyester sind Polylactide. Weiterhin bevorzugt sind Copolyester, die ausgewählt sind aus folgenden Gruppen: Polylactide, welche aus verschiedenen stereoisomeren Lactiden erhältlich sind, insbesondere Copolyester aus L-Lactid und D,L-Lactid, Copolyester aus Lactid und Glycolid, wobei das Lactid vorzugsweise D,L-Lactid und/oder L-Lactid darstellt.

Die nach dem erfindungsgemäßen Verfahren hergestellten Polyester weisen in der Regel eine mittlere inhärente Viskosität (Ubbelohde-Viskosimeter, Chloroform, 0.1%, 25°C) im Bereich von etwa 0.20 bis 5.0 dl/g, vorzugsweise im Bereich von etwa 0.50 bis 3.0 dl/g, auf. Diese Polyester weisen daher entsprechend hohe Molekulargewichte auf, die vorzugsweise eine Untergrenze von etwa 40.000 g/Mol (Dalton) aufweisen.

In der Regel bedeutet ein höheres Molekulargewicht, dass der Abbau des Polyesters langsamer abläuft als bei niedrigerem Molekulargewicht, da für den vollständigen Abbau mehr Bindungen zersetzt werden müssen. Die Polyester der vorliegenden Erfindung werden daher eher langsamer resorbiert als Polyester mit niedrigerem Molekulargewicht.

Die erfindungsgemäßen resorbierbaren Polyester verfügen über geeignete Resorptions-Charakteristika, d.h. diese sind bei der Herstellung und gegebenenfalls Weiterverarbeitung sowie bei Gebrauch stabil. Die Polyester können nach der Herstellung beispielsweise mehrere Jahre gelagert werden; erst bei Einsatz im/am Körper kommt es zur Resorption. Bei Einsatz in Form von pharmazeutischen Formulierungen hängt die Freisetzung häufig maßgeblich vom Abbau des verwendeten Polyesters ab, so dass mit der Abbauzeit auch die Freisetzung eines Wirkstoffs in gewünschter Weise gesteuert werden kann.

Der Begriff "resorbierbar" (lateinisch *resorbere* = "aufsaugen"), "Resorption" oder "abbaubar" oder "Abbau" im Zusammenhang mit einem erfindungsgemäßen Polymer, wie hier verwendet, bezeichnet ein Polymer, dessen molekulare Struktur in kleinere Einheiten zersetzt werden kann, ungeachtet des Abbaumechanismus. Der Begriff "Resorption" soll im Rahmen der Erfindung ganz allgemein als Stoffaufnahme in biologischen Systemen verstanden werden. Hierbei soll nicht zwischen passiver und aktiver Resorption unterschieden werden. Beispielsweise wird ein resorbierbares Polymer durch Hydrolyse der Esterbindung abgebaut bzw. zersetzt, wobei Wasser derart mit dem Polymer reagiert, dass aus dem Polymer kleinere, nicht-polymere Einheiten bis hin zu Monomeren gebildet werden. Die erfindungsgemäßen resorbierbaren Polymere sind daher hydrolytisch resorbierbar, was bedeutet, dass chemische Bindungen, im vorliegenden Fall die Esterbindung, im Polymer vorliegen, welche durch Wasser spaltbar sind, woraus kleinere Struktureinheiten resultieren.

Der Begriff "bioabbaubar" bezeichnet erfindungsgemäß im weitesten Sinne alle Materialien, die durch Mikroorganismen oder Enzyme z.B. im Körper abgebaut werden. In Zusammenhang mit einem erfindungsgemäßen Polymer, wie hier verwendet, bedeutet der Begriff "bioabbaubar", dass ein Polymer unter Einfluss von Wasser und ein oder mehreren Enzymen zu kleineren Einheiten abgebaut bzw. zersetzt wird. Beispielsweise wird Polymilchsäure durch Hydrolyse in Milchsäure zersetzt, die durch eine Vielzahl von Enzymen unter enzymatischer Zersetzung einem Bioabbau unterliegt.

Weiterhin können die Polyester der Erfindung für den tierischen und menschlichen Körper zugelassene Hilfsstoffe enthalten.

Die resorbierbaren Polyester der Erfindung sind auch mit anderen abbaubaren Monomeren kombinierbar.

Gegenstand der vorliegenden Erfindung ist auch die Verwendung der hochmolekularen, resorbierbaren Polyester, die gemäß dem erfindungsgemäßen Verfahren hergestellt werden, im pharmazeutischen oder medizinischen Bereich, insbesondere zur Herstellung pharmazeutischer Freigabesysteme.

Ein weiterer Aspekt der vorliegenden Erfindung stellt die Verwendung der hochmolekularen, resorbierbaren Polyester dar, die gemäß dem erfindungsgemäßen Verfahren hergestellt werden, insbesondere zur Herstellung von resorbierbaren Implantaten zum Einsatz im menschlichen oder tierischen Körper, für Folien, Membranen sowie pharmazeutische Formulierungen, insbesondere kontrollierte Freisetzungssysteme aller Art.

Nachfolgend wird die vorliegende Erfindung anhand von Figuren erläutert, welche die erfindungsgemäße Lehre veranschaulichen, diese aber nicht beschränken sollen.

### KURZE BESCHREIBUNG DER ZEICHNUNGEN

- Figur 1: veranschaulicht in einem Ablaufschema anhand einer beispielhaften Ausführungsform die Durchführung der Polymerisation gemäß dem erfindungsgemäßen Verfahren (Verfahrensschritte (a) bis (f));
- Figur 2: veranschaulicht in einem Ablaufschema anhand einer beispielhaften Ausführungsform die Durchführung der Reinigung des Rohpolymers durch Fällung gemäß dem erfindungsgemäßen Verfahren (Verfahrensschritte (g) bis (i)) und
- Figur 3: veranschaulicht in einem Ablaufschema anhand einer beispielhaften Ausführungsform die Durchführung der Trocknung, gegebenenfalls nach Zerkleinern des Polymers, gemäß dem erfindungsgemäßen Verfahren (Verfahrensschritte (j) und (k)).

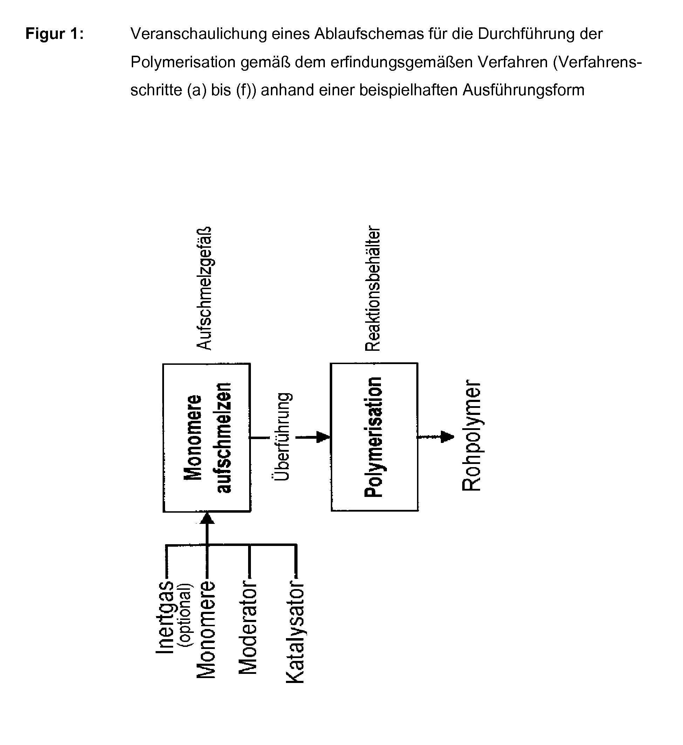

Im Einzelnen zeigt Figur 1 in einem Ablaufschema anhand einer beispielhaften Ausführungsform die Durchführung der Polymerisation gemäß dem erfindungsgemäßen Verfahren (Verfahrensschritte (a) bis (f)). Hierzu werden zunächst die Monomeren in einem Aufschmelzgefäß, vorzugsweise unter inerter Atmosphäre, geschmolzen (Verfahrensschritt (a)). Der Ansatz kann in kleinem (z.B. Labor-Maßstab) oder großem (z.B. großindustriellem) Maßstab gewählt werden.

Beispielsweise kann als Reaktionsgefäß ein Doppelmantelreaktor eingesetzt werden, der, vorzugsweise unter Stickstoffbegasung, mit einer entsprechenden Menge cyclischer Monomeren befüllt wird. Die Menge wird abhängig vom Durchführungsmaßstab gewählt, beispielsweise von einigen Gramm bis zu einigen Kilogramm Monomere. Der Doppelmantel des Reaktors wird dann zum Aufschmelzen der Monomeren auf eine erhöhte Temperatur, z.B. 100 bis 160°C Außentemperatur, aufgeheizt.

Sobald die Monomeren, beispielsweise D,L-Lactid, vollständig geschmolzen sind, werden ein Kettenlängenmoderator und ein Katalysator zugegeben (Verfahrensschritte (b) und (c)). Der Katalysator wird immer erst nach dem Schmelzen der Monomeren zugesetzt. Der Kettenlängenmoderator kann vor, während oder nach erfolgtem Aufschmelzen der Monomeren zugegeben werden. Ansonsten ist die Zugabereihenfolge von Moderator und Katalysator nicht kritisch. Beide können auch gleichzeitig zugegeben werden. Die Zugabe kann als reine Substanz oder gelöst in einem Lösungsmittel erfolgen. Die Reihenfolge der Verfahrensschritte (b) und (c) kann auch vertauscht werden, die Zugabe des Kettenlängenmoderators erfolgt dann vor Zugabe des Katalysators. Der Kettenlängenmoderator kann aber auch bereits zu den Monomeren in das Aufschmelzgefäß gegeben und zusammen mit den Monomeren geschmolzen werden. Nach einer weiteren bevorzugten Ausführungsform der Erfindung kann der Katalysator auch im Kettenlängenmoderator gelöst und beide können zusammen nach dem Aufschmelzen der Monomeren zur Reaktion zugegeben werden.

In Figur 1 werden gemäß einer bevorzugten Ausführungsform des erfindungsgemäßen Verfahrens der Kettenlängenmoderator und der Katalysator zusammen zu den bereits geschmolzenen Monomeren zugegeben, der Katalysator kann beispielsweise in einem Lösungsmittel, wie Toluol, gelöst zugesetzt werden. Der Kettenlängenmoderator ist beispielsweise Äpfelsäure oder Zitronensäure; der Katalysator ist zum Beispiel Zinn(II)-2-ethylhexanoat, gelöst in Toluol.

Die Katalysatormenge ist dabei so berechnet, dass der Zinngehalt bezogen auf die gesamte Reaktionsmasse vorzugsweise so gering wie möglich eingestellt ist, z.B. 10 bis 50 ppm beträgt.

Zur Homogenisierung der erhaltenen Reaktionsmischung, die im Wesentlichen aufgebaut ist aus Monomeren, Katalysator und Kettenlängenmoderator, wird in der Regel durchmischt, vorzugsweise gerührt (Verfahrensschritt (d)). Die Dauer bis zum Erhalt einer homogenen Mischung hängt von den eingesetzten Ausgangsmaterialien ab und kann wenige Minuten, beispielsweise etwa 2 bis etwa 10 Minuten, dauern.

Anschließend wird die nun homogene Mischung vorzugsweise unter Inertgas, wie Stickstoffbegasung, in den oder die Reaktionsbehälter überführt (Verfahrensschritt (e)). Dies kann in einfacher Weise durch Ablassen der Mischung aus dem Aufschmelzgefäß über ein oder mehrere Leitungen, beispielsweise ein oder mehrere Schläuche, mittels Schwerkraft, durch Pumpen, Einsatz von Inertgas unter Druck oder in beliebiger anderer Weise durchgeführt werden. Die Zahl der Reaktionsbehälter hängt vom gewählten Ansatz ab. Zum Beispiel können 1 bis 150 Behälter, bevorzugt 2 bis 100 Behälter, ganz besonders bevorzugt 2 bis 60 Behälter verwendet werden. Diese Reaktionsbehälter sind vorzugsweise aus Kunststoff, ganz besonders bevorzugt können Kunststoffflaschen aus Polypropoylen eingesetzt werden. Die Reaktionsbehälter sind in der Regel volumenmäßig kleiner dimensioniert als das Aufschmelzgefäß. Beispielsweise können Nennvolumen im Bereich von etwa 0.5 bis 2 Liter zum Einsatz kommen. Die Behälter, vorzugsweise Flaschen, werden nach dem Befüllen verschlossen und die Polymerisation durchgeführt (Verfahrensschritt (f)). Die sich anschließende Polymerisation erfolgt üblicherweise bei einer Temperatur, die vorzugsweise im Bereich von etwa 100°C bis 160°C liegt. Gemäß einer besonders bevorzugten Ausführungsform werden die Reaktionsbehälter in einen auf etwa 100°C bis 160°C vorgeheizten Wärmeschrank gestellt und dort über einen Zeitraum von etwa 2 bis 8 Tagen belassen. Während dieser Zeit wird der Wärmeschrank mit einem leichten Stickstoffstrom inertisiert. Nach Entnahme aus dem Wärmeschrank werden die Behälter auf Raumtemperatur abgekühlt. Anschließend werden die entstandenen Rohpolymere den Behältern entnommen. Es können Materialproben genommen und hinsichtlich der inhärenten Viskosität sowie des Restgehaltes an Monomer mittels GC analysiert werden.

Je nach dem erwünschten Polymerisationsgrad kann die Polymerisation auch abgebrochen werden und es kann bis zur gewünschten Umsetzung polymerisiert werden.

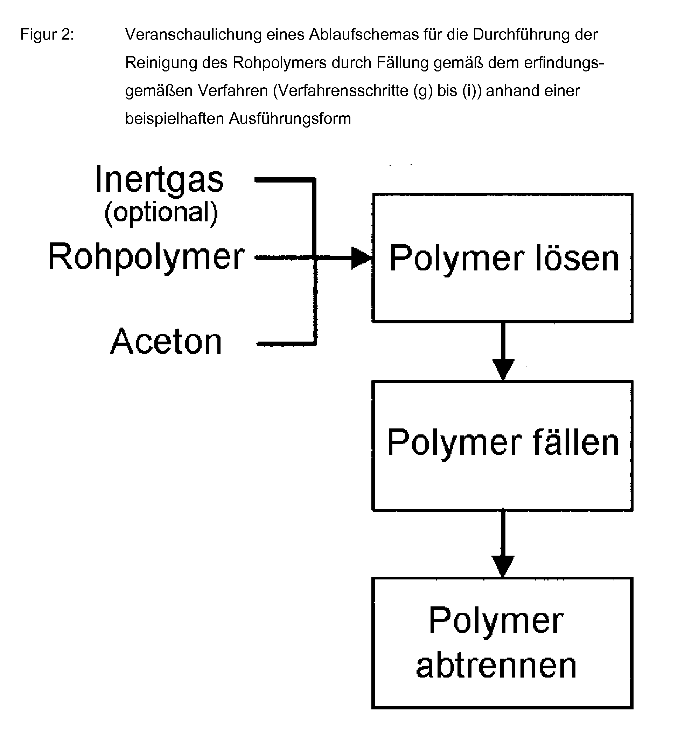

Das in Figur 2 im Einzelnen dargestellte Ablaufschema, welches die Reinigung durch Fällung gemäß dem erfindungsgemäßen Verfahren anhand einer beispielhaften Ausführungsform darstellt, schließt sich in der Regel unmittelbar an die Polymerisation gemäß dem Schema von Figur 1 an (Verfahrensschritte (g) bis (i)). Das erhaltene Rohpolymer kann aber auch zunächst gelagert und zu einem späteren Zeitpunkt gereinigt werden.

Gemäß Figur 2 wird das aus der Polymerisation erhaltene Rohpolymer zunächst in Aceton gelöst (Verfahrensschritt (g)). Vor der Ausfällung kann die Lösung durch Filtrieren gereinigt werden (nicht gezeigt). Die Ausfällung des gelösten Polymers erfolgt durch die Zugabe eines Überschusses an Wasser, vorzugsweise entmineralisiertes Wasser, wodurch die verbliebenen Monomeren in Lösung bleiben und somit aus dem Polymer entfernt werden können (Verfahrensschritt (h)). Das erhaltene Polymer kann anschließend, auch mehrfach, mit Wasser gewaschen werden. Die Abtrennung des gereinigten Polymers (Verfahrensschritt (i)) aus der Lösung kann üblicherweise durch Filtrieren, Absaugen oder dergleichen erfolgen.

Die Menge an Aceton zum Lösen des Rohpolymers sowie die Menge an Wasser zum Ausfällen hängen von der Art, dem Aufbau sowie der Menge des eingesetzten Rohpolymers ab. Der Fachmann kann diese Mengen durch einige orientierende Versuche ohne weiteres bestimmen.

Die Reinigung durch Lösen/Fällen/Abtrennen kann mehrfach wiederholt werden.

Das erhaltene gereinigte Polymer weist einen deutlich verringerten Restmonomergehalt auf, der in der Regel unterhalb von etwa 0.7 Gew.-%, bevorzugt unterhalb von etwa 0.5 Gew.-%, teilweise sogar unterhalb der Nachweisgrenze liegt.

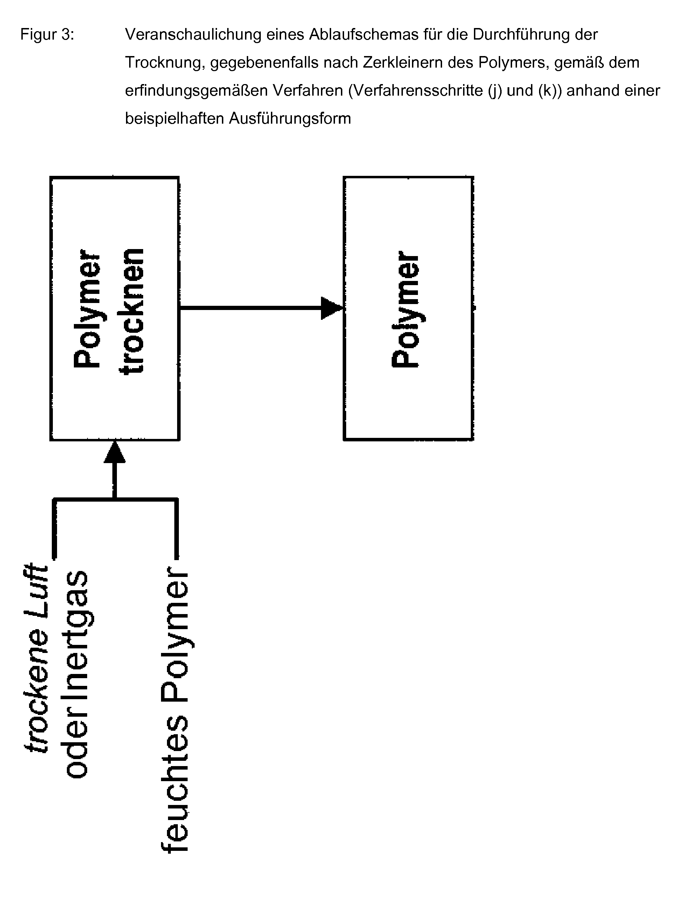

In Figur 3 ist in einem Ablaufschema anhand einer beispielhaften Ausführungsform die Trocknung, gegebenenfalls nach einem entsprechenden Zerkleinern des Polymers, gemäß dem erfindungsgemäßen Verfahren (Verfahrensschritt ((j) und (k)) dargestellt. Figur 3 schließt sich in der Regel unmittelbar an die Fällung gemäß dem in Figur 2 dargestellten Schema an, kann aber auch zu einem späteren Zeitpunkt durchgeführt werden. Das feuchte Polymer wird in einem üblichen Verfahren getrocknet.

Die Figuren 1 bis 3 verdeutlichen mögliche Ausführungsformen, sind jedoch nicht abschließend und damit auch nicht beschränkend im Hinblick auf den Schutzbereich des erfindungsgemäßen Verfahrens. Andere Verfahrensvarianten im Rahmen der Ansprüche sind je nach gewünschtem Eigenschaftsprofil denkbar.

Die nachfolgenden Beispiele dienen der Illustration exemplarisch durchgeführter Verfahren zur Herstellung der resorbierbaren Polyester. Sie sind lediglich als mögliche, beispielhaft dargestellte Vorgehensweisen zu verstehen, ohne die Erfindung auf deren Inhalt zu beschränken.

### AUSFÜHRUNGSBEISPIELE

### Beispiel 1

### Poly(D,L-lactid-co-glycolid)

D,L-Lactid und Glycolid werden im gewünschten Molmassenverhältnis (hier 76:24 Mol %) zusammen mit 1700 ppm Milchsäure aufgeschmolzen. Zum Starten der Reaktion werden 100 ppm Zinn(II)-Salz (typischerweise in der Form von Zinn(II)-2-ethylhexanoat) zugegeben. Die Reaktionsmasse wird nach der Homogenisierung auf etwa 1 I fassende Gebinde vereinzelt und die Reaktion erfolgt als Massepolymerisation bei 112°C über drei Tage.

Das Rohpolymer weist folgende Analysenwerte auf:
Inhärente Viskosität (i.V.) (CHCl₃, 25°C, 0.1 %): 0.75 dl/g
Restmonomergehalt D,L-Lactid (GC): 1.5 Gew.-%

Das entstandene Rohpolymer wird in Aceton gelöst und durch Zugabe von Wasser gefällt um nicht umgesetzte Monomere zu entfernen. Im Anschluss an die Reinigung wird das feuchte Polymer getrocknet.

Das Polymer hat eine i.V. von 0.81 dl/g und einen Restmonomergehalt an Lactid von 0.05 Gew.-%.

### Beispiel 2

### Poly(D,L-lactid-co-glycolid)

Es wird wie in Beispiel 1 vorgegangen, jedoch wird hier das D,L-Lactid und Glycolid in einem Molverhältniss von 51:49 Mol % zusammen mit 1470 ppm Milchsäure aufgeschmolzen. Zum Starten der Reaktion werden nur 40 ppm Zinn(II)-salz (typischerweise in der Form von Zinn(II)-2-ethylhexanoat) zugegeben. Die Reaktion erfolgt als Massepolymerisation bei 150°C über fünf Tage.

Das Rohpolymer weist folgende Analysenwerte auf:
Inhärente Viskosität (i.V.) (CHCl₃, 25°C, 0.1 %): 0.67 dl/g
Restmonomergehalt D,L-Lactid (GC): 0.8 Gew.-%

Das entstandene Rohpolymer wird in Aceton gelöst und durch Zugabe von Wasser gefällt um nicht umgesetzte Monomere zu entfernen. Im Anschluss an die Reinigung wird das feuchte Polymer getrocknet.

Das Polymer hat einen Restmonomergehalt an Lactid von 0.05 Gew.-%.

### Beispiel 3

### Poly(D,L-lactid-co-glycolid)

Ein analog zu Beispiel 1 hergestelltes Polymer jedoch mit 5000 ppm Milchsäureethylester und 10 ppm Zinn(II)-salz (typischerweise in der Form von Zinn(II)-2-ethylhexanoat) wird hergestellt. Die Reaktion erfolgt als Massepolymerisation bei 150°C über fünf Tage.
Inhärente Viskosität (CHCl₃, 25°C, 0.1 %): 0.55 dl/g
Restmonomergehalt D,L-Lactid (GC): 1.3 Gew.-%

Das entstandene Rohpolymer wird in Aceton gelöst und durch Zugabe von Wasser gefällt um nicht umgesetzte Monomere zu entfernen. Im Anschluss an die Reinigung wird das feuchte Polymer getrocknet.

Das Polymer hat einen Restmonomergehalt an Lactid unterhalb der Nachweisgrenze.

## Patentansprüche

1. Verfahren zur Herstellung von resorbierbaren Polyestern, umfassend die Schritte
(a) Aufschmelzen von Monomeren in einem Aufschmelzgefäß;
(b) Zugabe eines Katalysators;
(c) Zugabe eines Kettenlängenmoderators, der ausgwählt ist aus der Gruppe bestehend aus Hydroxydicarbonsäuren und Hydroxytricarbonsäuren;
(d) Homogenisieren der Reaktionsmischung;
(e) Überführen der Reaktionsmischung in ein oder mehrere Reaktionsbehälter;
(f) Durchführen der Polymerisationsreaktion in den ein oder mehreren Reaktionsbehältern bis zum erwünschten Umsetzungsgrad der Polymerisation;
(g) Lösen des erhaltenen Rohpolymers in Aceton;
(h) Ausfällen des Polymers durch Zugeben von Wasser;
(i) Abtrennen des erhaltenen Polymers;
(j) gegebenenfalls Zerkleinern des erhaltenen Polymers und
(k) Trocknen des Polymers.

2. Verfahren nach Anspruch 1, dadurch gelennzeichnet, dass der Kettenlängenmorderator ausgewählt ist aus der Gruppe bestehend aus Äpfelsäure und Zitronensäure.

3. Verfahren nach Anspruch 1 oder 2, **dadurch gekennzeichnet, dass** Verfahrensschritt (a), Verfahrensschritt (d) und/oder Verfahrensschritt (g) unter Durchmischen, vorzugsweise unter Rühren, aber Verfahrensschritt (f) nicht unter Durchmischen durchgeführt werden.

4. Verfahren nach Anspruch 1, 2 oder 3, **dadurch gekennzeichnet, dass** das Überführen der Reaktionsmischung gemäß Schritt (e) aus dem Aufschmelzgefäß durch Leiten der Reaktionsmischung vom Aufschmelzgefäß in ein oder mehrere Reaktionsbehälter durchgeführt wird, wie durch konventionelles Pumpen, unter Verwendung von Schwerkraft oder durch Druckaufgabe mittels Inertgas, insbesondere Stickstoff.

5. Verfahren nach Anspruch 1, 2, 3 oder 4, **dadurch gekennzeichnet, dass** das Überführen der Reaktionsmischung gemäß Schritt (e) aus dem Aufschmelzgefäß durch Leiten der Reaktionsmischung vom Aufschmelzgefäß in ein oder mehrere Reaktionsbehälter unter Zwischenschalten eines Schmelzefilters, vorzugsweise ausgewählt aus Kunststoff oder Edelstahl, durchgeführt wird.

6. Verfahren nach Anspruch 1, 2, 3, 4 oder 5, **dadurch gekennzeichnet, dass** der Katalysator nach dem Aufschmelzen der Monomeren und der Kettenlängenmoderator vor, während oder nach dem Aufschmelzen der Monomeren zugegeben wird.

7. Verfahren nach Anspruch 1, 2, 3, 4, 5 oder 6, **dadurch gekennzeichnet, dass** die Reaktionsbehälter hinsichtlich ihres Volumens kleiner dimensioniert ausgewählt werden als das Aufschmelzgefäß, bevorzugt das Verhältnis des Innenvolumens des Aufschmelzgefäßes zum Innenvolumen eines der Reaktionsbehälter im Bereich von etwa 200 : 1 bis etwa 2:1, insbesondere im Bereich von etwa 100 : 1 bis etwa 2 : 1 ausgewählt wird.

8. Verfahren nach Anspruch 1, 2, 3, 4, 5, 6 oder 7, **dadurch gekennzeichnet, dass** das Material der Reaktionsbehälter ausgewählt wird aus ein oder mehreren Kunststoffen, besonders bevorzugt Kunststoffen aus der Gruppe der Polyolefine, Polycarbonate, fluorierten oder teilfluorierten Kunststoffen, ganz besonders bevorzugt Polypropylen, Polytetrafluorethan und Polymethylpenten.

9. Verfahren nach Anspruch 1, 2, 3, 4, 5, 6, 7 oder 8, **dadurch gekennzeichnet, dass** nach dem Lösen des erhaltenen Rohpolymers in Aceton gemäß Schritt (g) die Lösung zum Entfernen von Verunreinigungen filtriert wird.

10. Verfahren nach Anspruch 1, 2, 3, 4, 5, 6, 7, 8 oder 9, **dadurch gekennzeichnet, dass** das ausgefällte Polymer kontinuierlich aus der Lösung entfernt wird.

11. Verfahren nach Anspruch 1, 2, 3, 4, 5, 6, 7, 8, 9 oder 10, **dadurch gekennzeichnet, dass** die Monomere ausgewählt werden aus ein oder mehreren cyclischen Esterverbindungen, insbesondere Glycolid, Lactid, wie beispielsweise L-Lactid, D-Lactid, D,L-Lactid, meso-Lactid, beta-Propiolacton, gamma-Butyrolacton, delta-Valerolacton, epsilon-Caprolacton, 3-Methyl-1,4-dioxa-2,5-dion, p-Dioxanon, Morpholin-2,5-dion, Morpholin-2-on und Mischungen dieser, besonders bevorzugt L-Lactid, D-Lactid, D,L-Lactid, meso-Lactid und Glycolid sowie Mischungen dieser.

12. Verfahren nach Anspruch 1, 2, 3, 4, 5, 6, 7, 8, 9, 10 oder 11, **dadurch gekennzeichnet, dass** die Polymerisation gemäß Verfahrensschritt (f) in den Reaktionsbehältern bei einer Temperatur im Bereich von etwa 100°C bis etwa 160°C isotherm, vorzugsweise für mehrere Tage, durchgeführt wird.

13. Verfahren nach Anspruch 1, 2, 3, 4, 5, 6, 7, 8, 9, 10, 11 oder 12, **dadurch gekennzeichnet, dass** als Katalysator eine Zinnverbindung, bevorzugt Zinn(II)-2-ethylhexanoat, in einer Konzentration von etwa 200 ppm oder weniger, bevorzugt etwa 100 ppm oder weniger, bevorzugter etwa 75 ppm oder weniger, ganz besonders bevorzugt etwa 60 ppm oder weniger, insbesondere etwa 50 ppm oder weniger (jeweils berechnet als Zinn, bezogen auf die gesamte Reaktionsmasse) verwendet wird.

14. Resorbierbare Polyester, hergestellt nach einem Verfahren gemäß Anspruch 1, 2, 3, 4, 5, 6, 7, 8, 9, 10, 11, 12 oder 13.

15. Resorbierbare Polyester nach Anspruch 14, **dadurch gekennzeichnet, dass** diese ein Molekulargewicht mit einer Untergrenze von etwa 40.000 g/Mol (Dalton) aufweisen.

16. Verwendung der resorbierbaren Polyester nach Anspruch 14 oder 15 im pharmazeutischen oder medizinischen Bereich, insbesondere für pharmazeutische Formulierungen, pharmazeutische Freisetzungssysteme, resorbierbare Implantate, Folien oder Membranen.

17. Vorrichtung zur Durchführung des Verfahrens zur Herstellung von resorbierbaren Polyestern nach einem der Ansprüche 1 bis 14, umfassend
(a) ein Aufschmelzgefäß zum Aufschmelzen von Monomeren;
(b) eine Überführvorrichtung zum Überführen der Reaktionsmischung in ein oder mehrere Reaktionsbehälter;
(c) ein oder mehrere Reaktionsbehälter zum Durchführen der Polymerisationsreaktion bis zum erwünschten Umsetzungsgrad der Polymerisation;
(d) eine Vorrichtung zum Lösen in Aceton und Ausfällen des erhaltenen Rohpolymers durch Zugabe von Wasser;
(e) eine Vorrichtung zum Abtrennen des erhaltenen Polymers sowie
(f) eine Vorrichtung zum Trocknen des erhaltenen Polymers.
